# FASCICULE DE BREVET EUROPEEN

(11) **EP 0 574 317 B1**
(45) Date de publication et mention de la délivrance du brevet: **09.10.1996**
(21) Numéro de dépôt: 93401481.2
(22) Date de dépôt: 10.06.1993
(51) Int. Cl.: C07J 9/00, C07J 21/00, C07J 33/00, C07J 41/00

(54) **Nouveau procédé de préparation de composés stéroides 20-céto 21alpha-hydroxy et intermédiaires**
Neues Verfahren zur Herstellung 20-Keto, 21-alpha-Hydroxysteroide und Zwischenprodukte dazu
A new process for the preparation of 20-keto, 21-alpha-hydroxy steroids and intermediates therefor

(30) Priorité: 11.06.1992 FR 9207047
(43) Date de publication de la demande: 15.12.1993
(73) Titulaire: ROUSSEL UCLAF, 93230 Romainville (FR)
(72) Inventeur: Buendia, Jean, F-94170 Le Perreux Sur Marne (FR); Crocq, Véronique, F-75003 Paris (FR); Masson, Christian, F-60250 Mouy (FR); Prat, Denis, F-93500 Pantin (FR); Vivat, Michel, F-77400 Lagny Sur Marne (FR)
(74) Mandataire: Vieillefosse, Jean-Claude

(56) Documents cités:
- EP-A- 7 823
- CH-A- 629 504
- FR-A- 2 149 302

## Description

La présente invention concerne un nouveau procédé de préparation de composés stéroïdes 20-céto 21α-hydroxy et des intermédiaires.

L'invention a ainsi pour objet un procédé de préparation des composés de formule (I) : 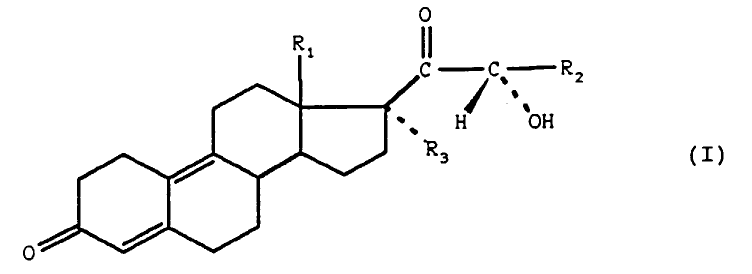 dans laquelle :
- R₁ représente un radical alkyle renfermant de 1 à 3 atomes de carbone,
- R₂ un radical alkyle renfermant de 1 à 12 atomes de carbone,
- R₃ un radical alkyle renfermant de 1 à 4 atomes de carbone,
caractérisé en ce que l'on traite un composé de formule (II) : 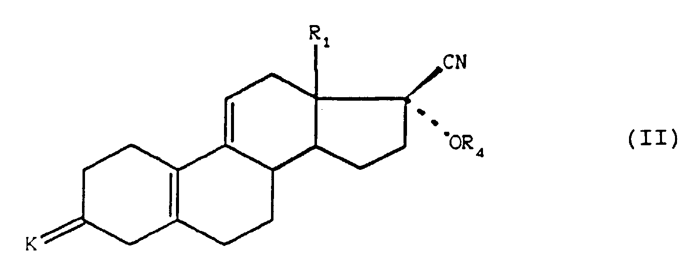 dans laquelle R₄ représente un reste d'éther facilement clivable, K représente une fonction cétone protégée sous forme de cétal, de thiocétal ou de cétal mixte et R₁ est défini comme précédemment, par un réactif organomagnésien de formule (A) :

HalMg-CH₂-R₂ (A)

dans laquelle Hal représente un atome d'halogène et R₂ est défini comme précédemment, pour obtenir un composé de formule (III) : 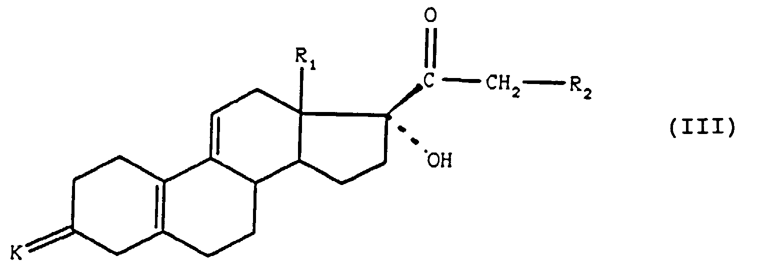 dans laquelle R₁, R₂ et K sont définis comme précédemment, que l'on traite par un agent d'acylation pour obtenir un composé de formule (IV) : 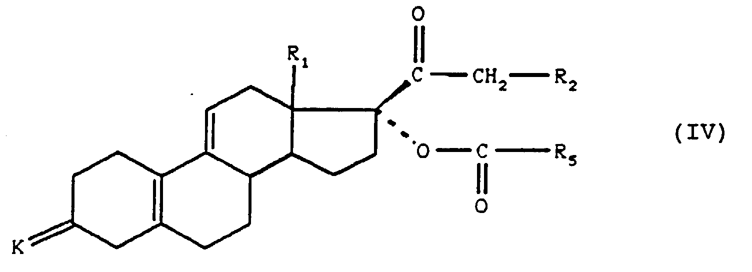 dans laquelle R₅ représente un radical alkyle renfermant de 1 à 6 atomes de carbone ou un radical aryle renfermant de 6 à 10 atomes de carbone et R₁, R₂ et K sont définis comme précédemment, que l'on soumet à l'action d'un agent d'alkylation approprié, pour obtenir un composé de formule (V) : 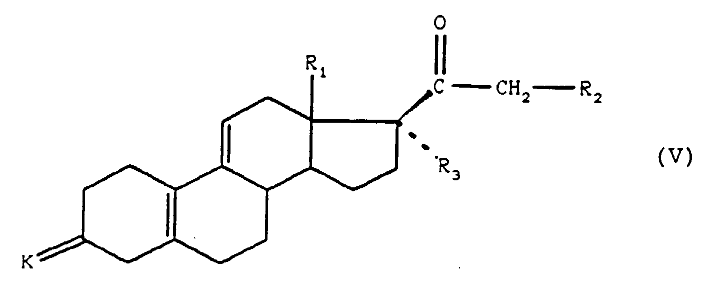 dans laquelle R₁, R₂, R₃ et K sont définis comme précédemment, que l'on soumet à une réaction d'autoxydation, pour obtenir un composé de formule (VI) : 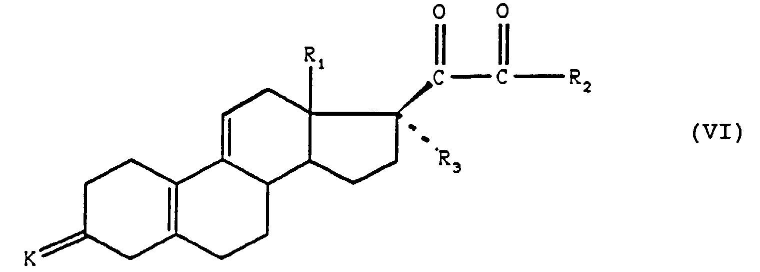 dans laquelle R₁, R₂, R₃ et K sont définis comme précédemment que l'on traite par un acide pour obtenir le composé 3-céto reconjugué de formule (VII) : 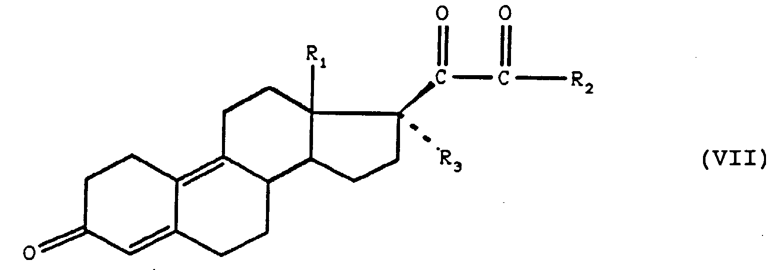 dans laquelle R₁, R₂ et R₃ sont définis comme précédemment, que l'on soumet à l'action d'un agent de réduction régio et énantiosélectif pour obtenir le composé de formule (I) attendu.

R₁ peut représenter un radical méthyle, éthyle ou propyle, méthyle étant préféré.

R₂ peut notamment représenter un radical méthyle, éthyle, propyle, isopropyle, n-butyle, isobutyle, n-pentyle, n-hexyle, 2-méthylpentyle, 2,3-diméthylbutyle, n-octyle ou 2,2-diméthylhexyle.

R₃ peut notamment représenter un radical méthyle, éthyle ou n-propyle.

K peut notamment représenter un groupement de formule : 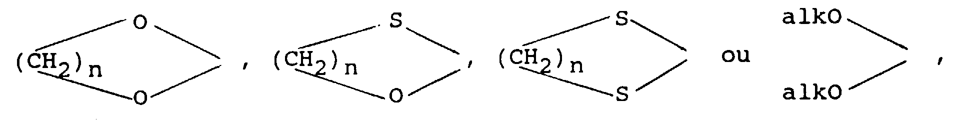 n étant égal à 2 ou 3 et alk représentant un radical alkyle renfermant de 1 à 4 atomes de carbone.

R₄ peut notamment représenter un radical alkyle renfermant de 1 à 6 atomes de carbone, par exemple méthyle, éthyle ou propyle, un radical tétrahydropyranyle, un radical aryle renfermant de 6 à 12 atomes de carbone, par exemple un radical phényle éventuellement substitué par 1 ou plusieurs radicaux méthyle, ou encore un radical silylé, par exemple trialkylsilyle tel que triméthyle ou diméthyl terbutylsilyle, triarylsilyle tel triphénylsilyle, ou diarylalkylsilyle tel que diphénylterbutylsilyle. La valeur trialkylsilyle et notamment triméthylsilyle est tout particulièrement préférée.

La réaction du composé de formule (II) avec le réactif magnésien de formule (A) est réalisée de préférence au sein d'un éther, par exemple l'éther éthylique, le tétrahydrofuranne, le dioxanne, ou au sein d'un solvant aromatique, par exemple le benzène ou le toluène, ou encore au sein d'un mélange de ces solvants. Dans la formule (A), Hal représente un atome de brome, de chlore ou d'iode.

L'hydrolyse conduisant au composé 17alpha-OH peut être effectuée par exemple par le chlorure d'ammonium, par un phosphate monoalcalin ou encore par un acide faible tel que l'acide acétique.

L'agent d'acylation que l'on fait agir sur le composé de formule (III) est de préférence un anhydride ou un halogénure d'acyle approprié. R₅ peut notamment représenter un radical méthyle, éthyle, propyle ou butyle, ou un radical phényle éventuellement substitué par un ou plusieurs radicaux méthyle. L'agent d'acylation est préférentiellement l'anhydride acétique ou le chlorure d'acétyle. On opère en présence d'une base qui peut être, par exemple, une base azotée telle que la pyridine, la diméthylaminopyridine ou encore la triéthylamine, au sein d'un solvant inerte tel que le benzène, le toluène, le xylène ou le cyclohexane ou en l'absence de solvant.

L'agent d'alkylation peut être un halogénure ou un sulfate d'alkyle, l'iodure étant plus particulièrement préféré. On effectue la réaction en présence d'un agent basique fort qui peut notamment être un amidure ou un alcoolate alcalin, à basse température au sein d'un solvant approprié anhydre, notamment un éther tel que le tétrahydrofuranne.

La réaction d'autoxydation consiste à faire réagir l'oxygène moléculaire sur un composé préalablement soumis à l'action d'une base forte qui transforme la fonction 20-céto en l'énolate correspondant. La base forte peut être notamment un alcoolate alcalin tel que le méthylate, l'éthylate ou le terbutylate de sodium ou de potassium ou le teramylate de sodium, un hydrure alcalin ou un amidure alcalin, par exemple de lithium, de sodium ou de potassium. L'oxygène est introduit de préférence par barbotage dans le milieu. On peut également opérer par barbotage d'air. On opère au sein d'un solvant organique qui peut être par exemple le diméthylformamide, le diméthylsulfoxyde, le diméthoxyéthane ....

Le déblocage de la cétone en position 3 peut selon la valeur de K, être effectué de différentes façons. On utilise un agent acide en milieu aqueux, dans le cas où K représente un cétal. Il s'agit par exemple d'un acide minéral ou organique tel que l'acide chlorhydrique, bromhydrique, sulfurique, perchlorique, nitrique, paratoluène sulfonique, acétique, formique, oxalique ou d'un mélange d'acides, ou encore d'une résine acide, par exemple une résine sulfonique. Dans le cas où K représente un thiocétal ou un cétal mixte, la déprotection de la fonction 3-oxo est réalisée par action de l'iode en présence d'une base par exemple un bicarbonate alcalin, ou par action de l'iode en quantité catalytique, en présence d'un agent oxydant, notamment l'eau oxygénée, par action de l'iodure de méthyle, de l'acide glyoxylique, ou encore de sels de métaux, tels le mercure ou le cadmium. On peut, en général, opérer dans un solvant tel qu'un alcanol inférieur, par exemple le méthanol ou l'éthanol, en mélange avec un solvant halogéné, par exemple le chlorure de méthylène, en présence d'eau. On obtient intermédiairement dans le cas d'un déblocage de thiocétal ou de cétal mixte, un produit de type 3-céto déconjugué de formule (VII') : 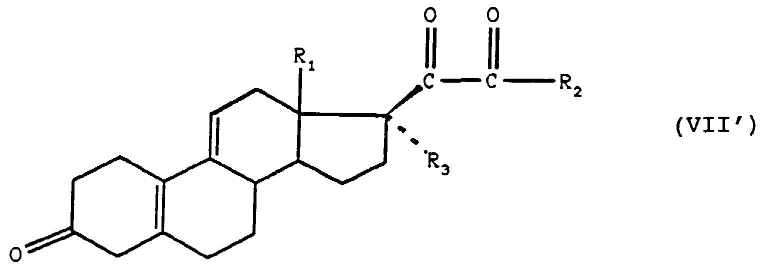 à partir duquel on obtient le dérivé de formule (VII) par traitement acide, dans les conditions indiquées ci-dessus.

La réduction régio et énantiosélective du composé de formule (VII) peut notamment être réalisée par voie enzymatique. Dans des conditions préférentielles de mise en oeuvre de ce procédé, la réduction est réalisée par l'action d'une levure, et notamment d'une levure choisie dans la classe des Hemiascomycetes et préférentiellement dans la famille des Saccharomycetaceae dont font partie les genres Saccharomyces, Kluyveromyces et Schizosaccharomyces ou Octosporomyces. La levure Saccharomyces cerevisiae est tout particulièrement préférée. La réaction peut être conduite au sein d'un solvant ou d'un mélange de solvants. Parmi ceux-ci, on peut citer notamment l'hexane, le toluène, les alcanols de 1 à 12 carbones, par exemple l'éthanol, l'isopropanol ou le dodécanol, le diméthylformamide, le diméthylsulfoxyde, le tétrahydrofuranne. On opère en présence d'un substrat carboné qui peut être notamment le glucose, le glycérol ou l'éthanol. On peut aussi opérer en présence d'un agent séquestrant du type cyclodextrine. Enfin, on opère à un pH qui est de préférence compris entre 3 et 7 et à une température qui peut se situer entre 25 et 50°C.

L'invention a notamment pour objet un procédé tel que décrit précédemment, caractérisé en ce que :
- l'on utilise au départ un composé de formule (II) dans laquelle R₄ représente un radical trialkylsilyle et notamment triméthylsilyle et K représente un groupement cétal et notamment éthylène cétal ;
- l'agent d'acylation que l'on fait agir sur le composé de formule (III) est l'anhydride acétique ou le chlorure ou le bromure d'acétyle ;
- l'agent d'alkylation est un halogénure ou un sulfate d'alkyle et l'on opère en présence d'un amidure ou d'un alcoolate alcalin ;
- la réaction d'autoxydation est effectuée par barbotage d'oxygène ou d'air sur un énolate en position 20 obtenu par action d'une base forte notamment un alcoolate ou un amidure alcalin, sur le composé de formule (V) ;
- le traitement acide du composé de formule (VI) dans laquelle K représente un groupement cétal est effectué par un mélange acide acétique-acide perchlorique ou chlorhydrique aqueux ;
- la réduction est effectuée par action d'une levure choisie dans le groupe cité plus haut et notamment de Saccharomyces cerevisiae.

L'invention a tout particulièrement pour objet un procédé tel que décrit précédemment, caractérisé en ce que l'on utilise au départ un composé de formule (II) dans laquelle R₁ représente un radical méthyle, un réactif de formule (A) dans laquelle R₂ représente un radical méthyle et, à titre d'agent d'alkylation, un agent de méthylation.

Une variante évidente du procédé, objet de la présente invention consiste à utiliser au départ un composé de formule (II') : 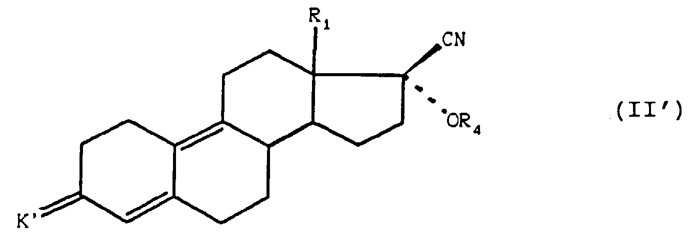 dans laquelle R₁ et R₄ sont définis comme précédemment et K' représente une fonction cétone protégée sous forme de thiocétal. On obtient ainsi les autres intermédiaires correspondants comportant un système de doubles liaisons identique à ci-dessus, puis après déblocage, le composé de formule (VII) puis le composé de formule (I).

L'invention a encore pour objet, à titre de composés industriels nouveaux les composés de formule (VIII) : 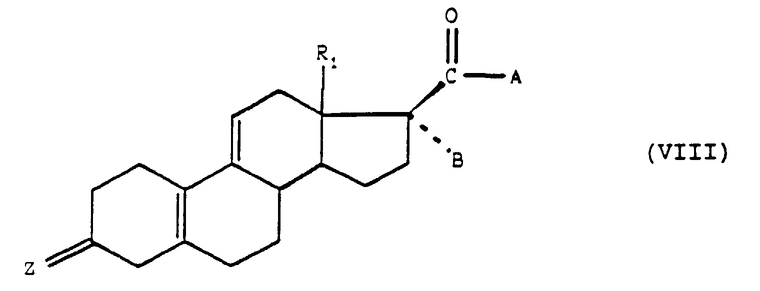 dans laquelle R₁ est défini comme ci-dessus et ou bien Z représente K, K étant défini comme ci-dessus, A représente -CO-R₂ et B représente R₃, R₂ et R₃ étant définis comme précédemment, ou bien Z représente un atome d'oxygène, A représente -CO-R₂ et B représente R₃, R₂ et R₃ étant définis comme ci-dessus ;
- les composés de formule (VII) telle que définie précédemment.

En dehors de leur fonction comme intermédiaires dans le cadre du procédé objet de la présente invention, les composés de formule (V) peuvent encore par déblocage de la fonction cétone en position 3, selon les méthodes décrites plus haut, conduire à certains composés du type de ceux décrits dans le brevet français 2 149 302 ou à des analogues, et notamment à la 17α-méthyl 17β-(1-oxopropyl) estra 4,9-dièn-3-one.

Les composés de formule (I) sont doués d'activités progestomimétique et anti-estrogène et sont décrits dans le brevet européen n° 7823.

Les composés de départ de formule (II) sont décrits dans le brevet français 2 082 129, ou peuvent être préparés par des procédés connus de l'homme du métier à partir de composés décrits dans ce brevet ou connus par ailleurs.

Les composés de formule (II') peuvent être obtenus par des procédés connus de l'homme du métier, à partir des composés 3-céto correspondants.

L'exemple suivant illustre l'invention sans toutefois la limiter.

### EXEMPLE : 17α-méthyl 17β-(2S-hydroxy 1-oxopropyl) estra 4,9-dièn-3-one

STADE A : 3-(1,2-éthanediyl) acétal cyclique de 17α-hydroxy 17β-(1-oxopropyl) estra 5(10), 9(11)-dièn-3-one
   On mélange sous gaz inerte 30 cm³ de tétrahydrofuranne anhydre et 20 cm³ d'une solution 3M de bromure d'éthylmagnésium dans l'éther éthylique. On ajoute lentement, à température ambiante 3,63 g de 3-(1,2-éthanediyl) acétal cyclique de 17α-triméthylsilyloxy 17β-cyano estra 5(10), 9(11) dièn-3-one en solution dans 10 cm³ de tétrahydrofuranne. On chauffe légèrement le milieu réactionnel pour éliminer l'éther éthylique et atteindre une concentration d'environt 2M du magnésien, puis on maintient le milieu réactionnel à +64°/+65°C pendant 16 heures. On refroidit à +15°/+20°C et verse dans le milieu, 25 cm³ d'une solution saturée de chlorure d'ammonium en contrôlant la température par un bain d'eau et de glace, puis à nouveau 40 cm³ d'une solution saturée de chlorure d'ammonium. On maintient sous agitation pendant 1 heure à 25°C puis extrait au chlorure de méthylène, lave la phase organique à l'eau, la sèche et évapore le solvant. On chromatographie le résidu sur silice en éluant au mélange toluène-acétate d'éthyle (85-15). On obtient 2,7 g de produit attendu que l'on recristallise dans l'éther isopropylique. F = 159°C.
   Spectre IR (CHCl₃)
   Absorptions à 3618 et 3513 cm⁻¹ (OH), 1706 et 1691 cm⁻¹ (C=O), 1640 et 1617 cm⁻¹ (C=C).
   Spectre RMN (CDCl₃, 300 MHz, ppm)
   0,23 (s), 0,66 (s) : 18-CH₃ ; 1,07 (t) : CH₃ du propyl ; 2,45-2,80 : - CH₂- du propyl ; 3,99 : cétal ; 5,57 : H₁₁.
STADE B : 3-(1,2-éthanediyl) acétal cyclique de 17α-acétoxy 17β-(1-oxopropyl) estra-5(10),9(11)-dièn-3-one
   On mélange sous gaz inerte 1,7 g de produit obtenu au stade A, 17 cm³ de toluène et 0,34 g de diméthylaminopyridine. On introduit lentement sous agitation 0,9 cm³ d'anhydride acétique et porte au reflux pendant 40 heures environ. On refroidit à 0°/+5°C et ajoute 10 cm³ d'acétate d'éthyle puis 34 cm³ d'une solution aqueuse saturée de chlorure d'ammonium. On agite pendant 1 heure à 0°/+5°C, décante et extrait la phase aqueuse à l'acétate d'éthyle. On lave les phases organiques réunies à l'eau, sèche et évapore le solvant. On reprend le résidu à l'éther isopropylique, essore les cristaux et les sèche. On obtient 1,98 g de produit attendu. F = 195°C.
   Spectre IR (CHCl₃)
   Absorptions à 1729-1715 cm⁻¹ (C=O), présence de cétal, absence d'OH.
   Spectre RMN (CDCl₃, 300 MHz, ppm)
   0,53 (s) : 18 CH₃ ; 1,05 (t) : CH₃ du propyl ; 2,07 (s) : O-AC ; 3,99 (cétal) ; 5,58 : H₁₁.
STADE C : 3-(1,2-éthanediyl) acétal cyclique de 17α-acétoxy 17β-(1-oxopropyl) estra-5(10) ,9(11)-dièn-3-one
   On mélange sous gaz inerte à -70°C, 8 cm³ d'ammoniac liquéfié et 33 mg de lithium et agite pendant 15 minutes. On introduit lentement 10 cm³ de tétrahydrofuranne anhydre puis agite à -75°C pendant 30 minutes et introduit 0,5 g de produit obtenu au stade B. On agite pendant 1 heure, puis ajoute lentement 0,375 cm³ d'iodure de méthyle et maintient sous agitation pendant 1 heure 30 minutes. On laisse ensuite remonter la température et élimine l'ammoniac, puis, vers 0°/+5°C, ajoute 25 cm³ d'eau et agite à +20°/+25°C pendant 30 minutes. On extrait à l'acétate d'éthyle, lave la phase organique à l'eau et sèche puis élimine le solvant. On chromatographie le résidu sur silice en éluant au mélange cyclohexaneacétate d'éthyle (91-9) et obtient après recristallisation dans l'éther isopropylique 0,34 g du produit attendu. F = 107°C.
   Spectre IR (CHCl₃)
   Absence d'acétate - Absorption à 1698 cm⁻¹ (cétone non conjuguée).
   Spectre RMN (CDCl₃, 300 MHz, ppm)
   0,60 (s) : 18-CH₃ ; 1,04 (t) : CH₃ du propyl ; 1,12 (s) : CH₃ en 17 ; 3,98 : cétal ; 5,58 : H₁₁.
STADE D : 3-(1,2-éthanediyl) acétal cyclique de 17α-méthyl 17β-(1,2-dioxo propyl) estra-5(10), 9(11)-dièn-3-one
   On mélange sous gaz inerte, 1,5 g de produit obtenu comme décrit au stade C et 15 cm³ de diméthylformamide. On agite à 20°C pendant 10 minutes puis refroidit à 0°/+5°C et ajoute 1 g de terbutylate de potassium. On agite la suspension à 0°/+5°C pendant 5 minutes puis refroidit à -25°C et introduit par barbotage 0,27 l d'oxygène. On maintient ensuite sous agitation en laissant remonter la température jusqu'à 0°C. On coule le mélange à 0°C, sous atmosphère inerte, sur 30 cm³ d'un tampon phosphate 0,2 M et agite pendant 30 minutes. On extrait à l'acétate d'éthyle, lave la phase organique à l'eau, sèche et évapore le solvant. On chromatographie le résidu sur silice en éluant au mélange toluène-chlorure de méthylène-acétate d'éthyle (90-7-3) et obtient, après recristallisation dans l'éther isopropylique, 1,07 g de produit attendu. F ∼ 100°C.
   Spectre IR (CHCl₃)
   Absorptions à 1725-1694 cm⁻¹ (C=O)
   Spectre RMN (CDCl₃, 300 MHz, ppm)
   0,66 (s) : 18-CH₃ ; 1,26 (s) : CH₃ en 17 ; 2,32 (s) : CH₃ du propyl ; 3,99 : cétal ; 5,59 : H₁₁.
STADE E :17α-méthyl 17β-(1,2-dioxo propyl) estra-4,9-dièn-3-one
   On mélange sous gaz inerte 0,2 g de produit obtenu au stade D et 1,5 cm³ d'acide acétique à 99,5 % puis ajoute à température ambiante 0,075 cm³ d'acide perchlorique à 65 %, puis 0,075 cm³ d'eau. On agite pendant 2 heures puis ajoute un mélange de 5,2 cm³ d'eau et 2,8 g de glace. On extrait au chlorure de méthylène, lave la phase organique à l'eau, la sèche et la concentre à sec. On chromatographie le résidu sur silice en éluant au mélange toluène-dioxane (95-5) et obtient 0,142 g de produit attendu.
   Spectre IR : (CHCl₃)
   Absorptions à 1716-1694 cm⁻¹ (C=O), 1653 cm⁻¹ (C=O conjuguée), 1607 cm⁻¹ (C=C).
   Spectre RMN : (CDCl₃ -300 MHz, ppm)
   0,86 : 18-CH₃ ; 1,26 : CH₃ en 17 ; 2,33 : CH₃ du propyl ; 5,68 : C=C.
STADE F :17α-méthyl 17β-(2S-hydroxy 1-oxo propyl) estra-4,9-dièn-3-one
   On mélange à 30°C ± 1°C, 500 cm³ de tampon acétate de sodium et 20 g de levure Saccharomyces cerevisiae. On agite pendant 20 minutes à 30°C puis ajoute 6,6 g de glucose. Tout en maintenant la température à 30°C, et le pH à 5,4 par addition de soude 2N ou d'acide acétique 2M, on introduit 0,1 g de produit obtenu au stade E en solution dans le diméthylsulfoxyde, puis maintient sous agitation pendant 22 heures. Après centrifugation du milieu pendant 45 minutes à 20 000 g et à 20°C maximum, on extrait le surnageant à l'acétate d'éthyle, sature à 50 % la phase aqueuse par addition de chlorure de sodium, puis extrait de nouveau à l'acétate d'éthyle. Les phases organiques réunies sont lavées à l'eau, séchées et concentrées à sec. On obtient 100,3 mg de produit attendu brut, dont une analyse par HPLC montre qu'il renferme 99,75 % d'isomère 21-OH(S).
   On chromatographie 0,090 g du produit brut sur silice en éluant au mélange toluène-acétate d'éthyle-isopropanol (90-6-4) et obtient 0,043 g de produit attendu. F = 115°C.
   Spectre RMN (CDCl₃ - 300 MHz, ppm).
   0,83 (s) : 18-CH₃ ; 1,18 (s) : CH₃ en 17 ; 1,32 (d) : CH₃-CH-
STADE F' : 17α-méthyl 17β-(2 S-hydroxy 1-oxo propyl) estra-4,9-dièn-3-one
   Des essais ont été réalisés avec les levures figurant sur le tableau ci-dessous, dans les conditions suivantes :
   a) La croissance de la levure a été effectuée sur un milieu de type synthétique (tampon phosphate + source d'azote minéral) complété en oligoéléments et vitamines, la source du carbone étant le glucose. On obtient un milieu de pH 6,4. L'incubation est faite à 30°C, sur agitateur orbital à 150 t/m pendant 24 heures.
   b) Pour effectuer la bioconversion, on dilue au préalable 1 volume de bouillon de croissance dans 1 volume de milieu neuf contenant une nouvelle source de carbone constituée par du glycérol, à raison de 40 g/l et une concentration en substrat dicétone de 1 g/l. Aucun tiers solvant n'est ajouté. On obtient un pH d'environ 5,5.

Le traitement des essais est effectué après des durées variables d'incubation, sous agitation magnétique et barbotage d'air, à 30°C environ. Pour cela on prélève 100 µl de mélange que l'on place dans un tube contenant des billes de verre, ajoute 5 ml de chlorobutane et agite pendant 1 minute. On récupère la phase supérieure et détermine par HPLC la quantité de produit attendu présent, que l'on rapporte au volume total de l'essai.

Les résultats figurent sur le tableau ci-après :

| Nom de la souche | N° MUCL * | Durée culture h | Produit obtenu en mg ** |
|---|---|---|---|
| Saccharomyces chevalieri | 27815 | 24 | 62 |
| Saccharomyces hienipiensis | 27820 | 24 | 82,5 |
| Saccharomyces italicus | 27822 | 24 | 75 |
| Saccharomyces uvarum | 27835 | 144 | 60 |
| Octosporomyces japonicus | 27840 | 24 | 65 |
| Octosporomyces octosporus | 27842 | 24 | 85 |
| Saccharomyces carlsbergensis | 28756 | 48 | 30 |
| Saccharomyces pastorianus | 29299 | 23 | 57 |
| Kluyveromyces thermotolerans | 28822 | 23 | 92,5 |
| Kluyveromyces marxianus | 27725 | 23 | 59 |
| Schizosaccharomyces pombe | 28824 | 23 | 100,5 |
| Saccharomyces cerevisiae | | 23 | 54,5 |

| | | | |
|---|---|---|---|
| * Souches de levures commercialisées sous ce n°, de la Mycothèque de l'Université Catholique de Louvain. | | | |
| ** Il s'agit ici du produit estimé au niveau du bouillon, que l'on peut purifier comme décrit au stade F. | | | |

## Revendications

1. Procédé de préparation des composés de formule (I) : 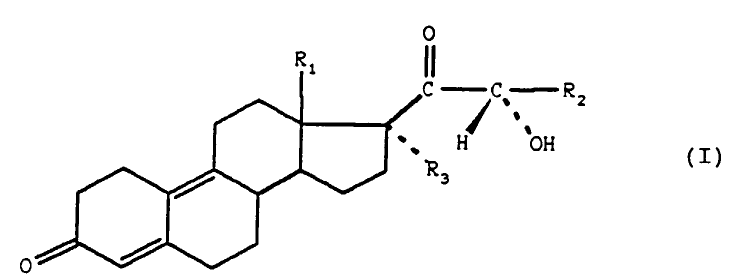 dans laquelle :
- R₁ représente un radical alkyle renfermant de 1 à 3 atomes de carbone,
- R₂ un radical alkyle renfermant de 1 à 12 atomes de carbone,
- R₃ un radical alkyle renfermant de 1 à 4 atomes de carbone,
caractérisé en ce que l'on traite un composé de formule (II) : 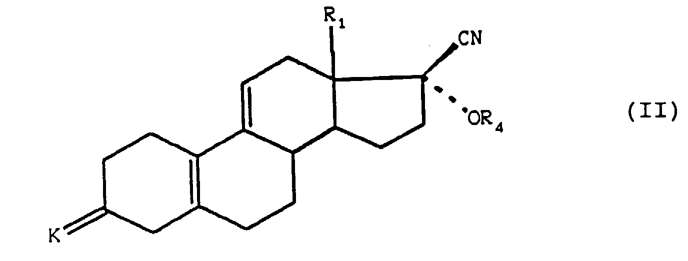 dans laquelle R₄ représente un reste d'éther facilement clivable, K représente une fonction cétone protégée sous forme de cétal, de thiocétal ou de cétal mixte et R₁ est défini comme précédemment, par un réactif organomagnésien de formule (A) :
HalMg-CH₂-R₂ (A)
dans laquelle Hal représente un atome d'halogène et R₂ est défini comme précédemment, pour obtenir un composé de formule (III) : 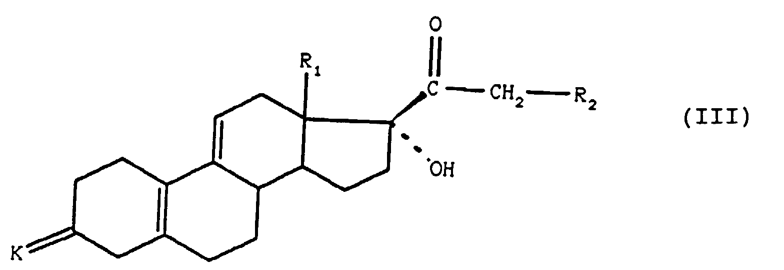 dans laquelle R₁, R₂ et K sont définis comme précédemment, que l'on traite par un agent d'acylation pour obtenir un composé de formule (IV) : 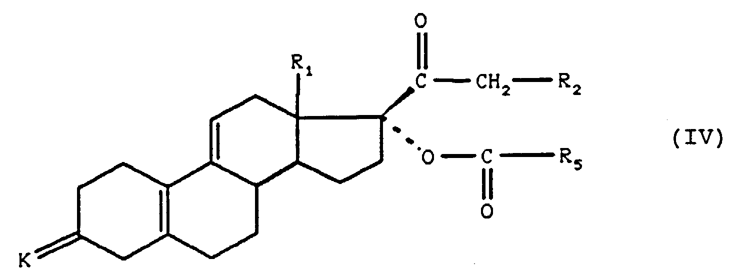 dans laquelle R₅ représente un radical alkyle renfermant de 1 à 6 atomes de carbone ou un radical aryle renfermant de 6 à 10 atomes de carbone et R₁, R₂ et K sont définis comme précédemment, que l'on soumet à l'action d'un agent d'alkylation approprié, pour obtenir un composé de formule (V) : 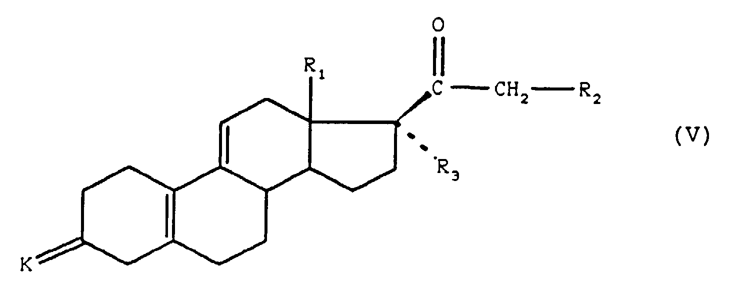 dans laquelle R₁, R₂, R₃ et K sont définis comme précédemment, que l'on soumet à une réaction d'autoxydation, pour obtenir un composé de formule (VI) : 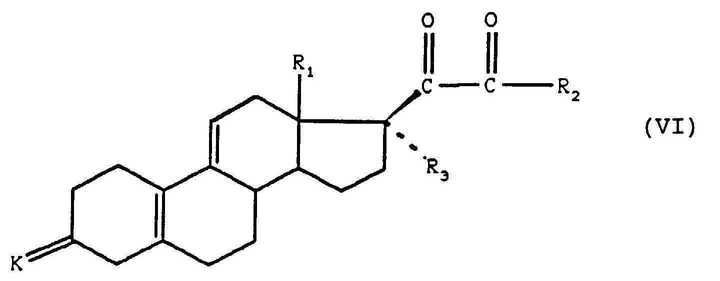 dans laquelle R₁, R₂, R₃ et K sont définis comme précédemment que l'on traite par un acide pour obtenir le composé 3-céto reconjugué de formule (VII) : 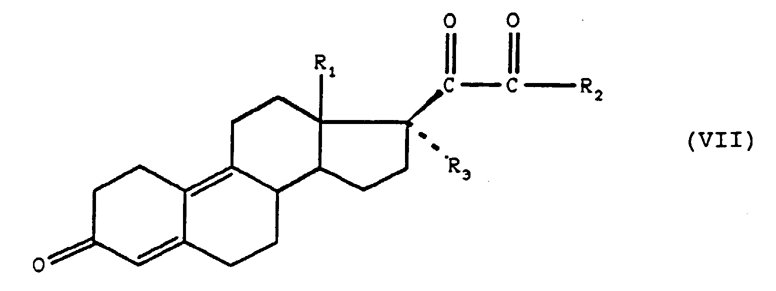 dans laquelle R₁, R₂ et R₃ sont définis comme précédemment, que l'on soumet à l'action d'un agent de réduction régio et énantiosélectif pour obtenir le composé de formule (I) attendu.

2. Procédé selon la revendication 1, caractérisé en ce que l'on utilise au départ un composé de formule (II) dans laquelle R₄ représente un radical alkyle renfermant de 1 à 6 atomes de carbone, un radical tétrahydropyranyle, un radical aryle renfermant de 6 à 12 atomes de carbone ou un radical silylé.

3. Procédé selon la revendication 2, caractérisé en ce que l'on utilise au départ un composé de formule (II) dans laquelle R₄ représente un radical trialkylsilyle et notamment triméthylsilyle et K représente un groupement cétal et notamment éthylène cétal.

4. Procédé selon l'une quelconque des revendications 1 à 3, caractérisé en ce que l'agent d'acylation est l'anhydride acétique ou le chlorure ou le bromure d'acétyle.

5. Procédé selon l'une quelconque des revendications 1 à 4, caractérisé en ce que l'agent d'alkylation est un halogénure ou un sulfate d'alkyle.

6. Procédé selon l'une quelconque des revendications 1 à 5, caractérisé en ce que la réaction d'autoxydation est réalisée par barbotage d'oxygène ou d'air sur un énolate en position 20 obtenu par action d'une base forte sur le composé de formule (V).

7. Procédé selon l'une quelconque des revendications 1 à 6, caractérisé en ce que la réduction est réalisée par voie enzymatique.

8. Procédé selon la revendication 7, caractérisé en ce que la réduction est réalisée à l'aide d'une levure.

9. Procédé selon la revendication 7 ou 8, caractérisé en ce que la réduction est réalisée à l'aide d'une levure choisie dans la classe des Hemiascomycetes.

10. Procédé selon la revendication 7, 8 ou 9, caractérisé en ce que la réduction est réalisée à l'aide de Saccharomyces cerevisiae.

11. Procédé selon l'une quelconque des revendications 1 à 10, caractérisé en ce que l'on utilise au départ un composé de formule (II) dans laquelle R₁ représente un radical méthyle, un réactif de formule (A) dans laquelle R₂ représente un radical méthyle et, à titre d'agent d'alkylation, un agent de méthylation.

12. A titre de composés industriels nouveaux, les composés de formule (VIII) : 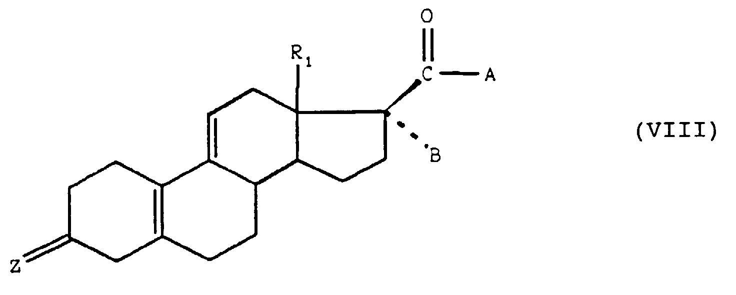 dans laquelle R₁ est défini comme à la revendication 1 et ou bien Z représente K, K étant défini comme à la revendication 1, A représente -CO-R₂ et B représente R₃, R₂ et R₃ étant définis comme à la revendication 1, ou bien Z représente un atome d'oxygène, A représente -CO-R₂ et B représente R₃, R₂ et R₃ étant définis comme à la revendication 1.

13. A titre de composés industriels nouveaux, les composés de formule (VII) telle que définie à la revendication 1.

## Patentansprüche

1. Verfahren zur Herstellung von Verbindungen der Formel (I) 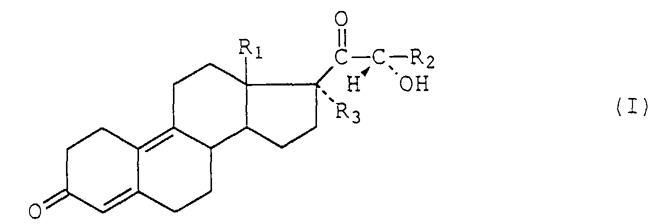 in der
- R₁ einen Alkylrest mit 1 bis 3 Kohlenstoffatomen darstellt,
- R₂ einen Alkylrest mit 1 bis 12 Kohlenstoffatomen bedeutet und
- R₃ einen Alkylrest mit 1 bis 4 Kohlenstoffatomen darstellt,
dadurch gekennzeichnet, daß man eine Verbindung der Formel (II) 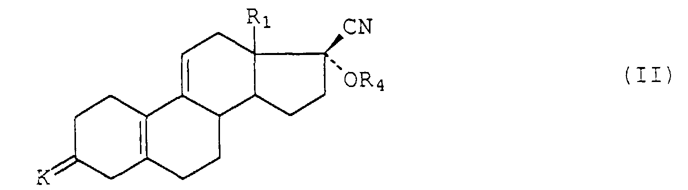 in der R₄ einen leicht abspaltbaren Etherrest darstellt, K eine in Form von Ketal, Thioketal oder gemischtem Ketal geschützte Ketonfunktion bedeutet und R₁ wie oben definiert ist, mit einem Organomagnesium-Reagens der Formel (A)
HalMg-CH₂-R₂ (A)
behandelt, worin Hal ein Halogenatom darstellt und R₂ wie oben definiert ist, um eine Verbindung der Formel (III) 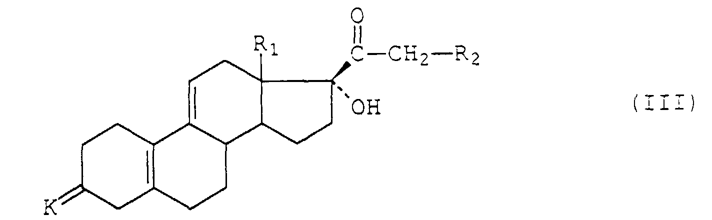 zu erhalten, in der R₁, R₂ und K wie oben definiert sind, die man mit einem Acylierungsmittel behandelt, um eine Verbindung der Formel (IV) 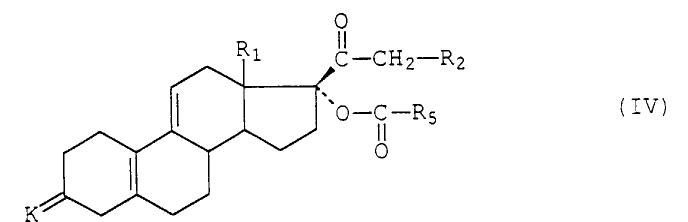 zu erhalten, in der R₅ einen Alkylrest mit 1 bis 6 Kohlenstoffatomen oder einen Arylrest mit 6 bis 10 Kohlenstoffatomen darstellt und R₁, R₂ und K wie oben definiert sind, die man der Einwirkung eines geeigneten Alkylierungsmittels unterzieht, um eine Verbindung der Formel (V) 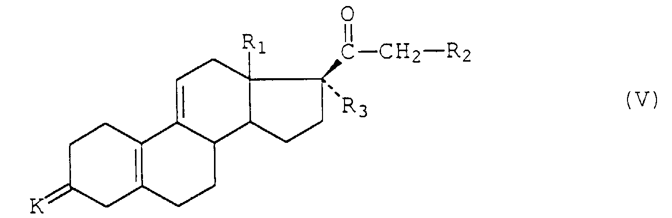 zu erhalten, in der R₁, R₂, R₃ und K wie oben definiert sind, die man einer Autoxydations-Reaktion unterwirft, um eine Verbindung der Formel (VI) 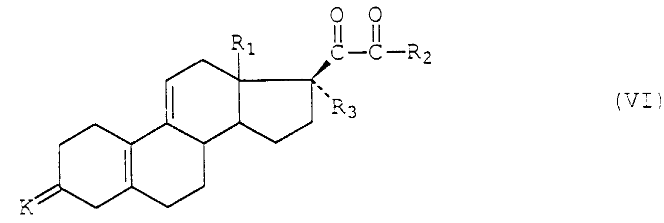 zu erhalten, in der R₁, R₂, R₃ und K wie oben definiert sind, die man mit einer Säure behandelt, um die rekonjugierte 3-Keto-Verbindung der Formel (VII) 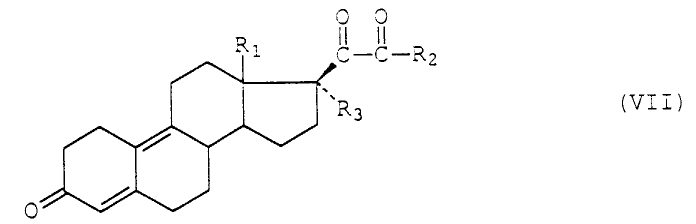 zu erhalten, in der R₁, R₂ und R₃ wie oben definiert sind, die man der Einwirkung eines regio- und enantioselektiven Reduktionsmittels unterzieht, um die erwartete Verbindung der Formel (I) zu erhalten.

2. Verfahren nach Anspruch 1, dadurch gekennzeichnet, daß man als Ausgangsprodukt eine Verbindung der Formel (II) verwendet, in der R₄ einen Alkylrest mit 1 bis 6 Kohlenstoffatomen, einen Tetrahydropyranylrest, einen Arylrest mit 6 bis 12 Kohlenstoffatomen oder einen Silylrest darstellt.

3. Verfahren nach Anspruch 2, dadurch gekennzeichnet, daß man als Ausgangsprodukt eine Verbindung der Formel (II) verwendet, in der R₄ einen Trialkylsilylrest und insbesondere Trimethylsilyl darstellt und K eine Ketalgruppe und insbesondere Ethylenketal bedeutet.

4. Verfahren nach irgendeinem der Ansprüche 1 bis 3, dadurch gekennzeichnet, daß das Acylierungsmittel Essigsäureanhydrid, Acetylchlorid oder Acetylbromid ist.

5. Verfahren nach irgendeinem der Ansprüche 1 bis 4, dadurch gekennzeichnet, daß das Alkylierungsmittel ein Alkylhalogenid oder ein Alkylsulfat ist.

6. Verfahren nach irgendeinem der Ansprüche 1 bis 5, dadurch gekennzeichnet, daß die Autoxydations-Reaktion durch Einleiten von Sauerstoff oder Luft in ein Enolat in Position 20, erhalten durch Einwirkung einer starken Base auf die Verbindung der Formel (V), realisiert wird.

7. Verfahren nach irgendeinem der Ansprüche 1 bis 6, dadurch gekennzeichnet, daß die Reduktion auf enzymatischem Wege realisiert wird.

8. Verfahren nach Anspruch 7, dadurch gekennzeichnet, daß die Reduktion mit Hilfe einer Hefe realisiert wird.

9. Verfahren nach Anspruch 7 oder 8, dadurch gekennzeichnet, daß die Reduktion mit Hilfe einer Hefe, gewählt aus der Klasse der Hemiascomyceten, realisiert wird.

10. Verfahren nach Anspruch 7, 8 oder 9, dadurch gekennzeichnet, daß die Reduktion mit Hilfe von Saccharomyces cerevisiae realisiert wird.

11. Verfahren nach irgendeinem der Ansprüche 1 bis 10, dadurch gekennzeichnet, daß man als Ausgangsprodukt eine Verbindung der Formel (II), in der R₁ einen Methylrest darstellt, ein Reagens der Formel (A), in der R₂ einen Methylrest bedeutet, und als Alkylierungsmittel ein Methylierungsmittel verwendet.

12. Als neue industrielle Verbindungen die Verbindungen der Formel (VIII) 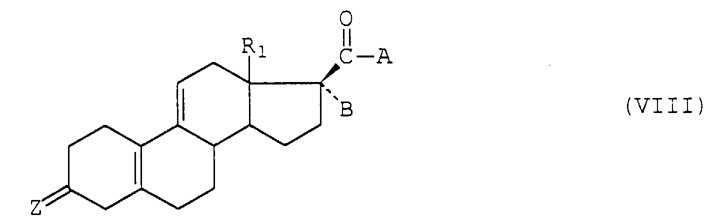 in der R₁ wie in Anspruch 1 definiert ist, und entweder Z die Bedeutung von K besitzt, wobei K wie in Anspruch 1 definiert ist, A -CO-R₂ darstellt und B R₃ bedeutet, wobei R₂ und R₃ wie in Anspruch 1 definiert sind, oder Z ein Sauerstoffatom darstellt, A -CO-R₂ bedeutet und B R₃ darstellt, wobei R₂ und R₃ wie in Anspruch 1 definiert sind.

13. Als neue industrielle Verbindungen die wie in Anspruch 1 definierten Verbindungen der Formel (VII).

## Claims

1. Preparation process for compounds of formula (I): 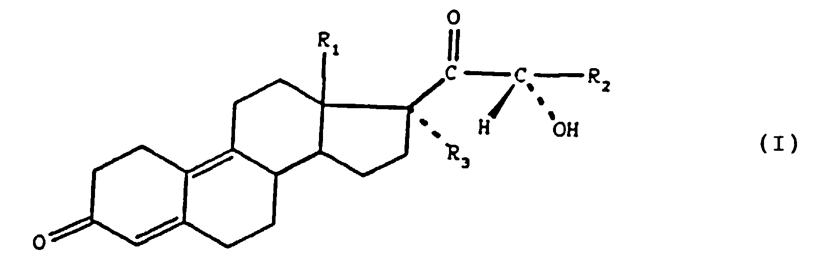 in which:
- R₁ represents an alkyl radical containing 1 to 3 carbon atoms,
- R₂ an alkyl radical containing 1 to 12 carbon atoms,
- R₃ an alkyl radical containing 1 to 4 carbon atoms,
characterized in that a compound of formula (II): 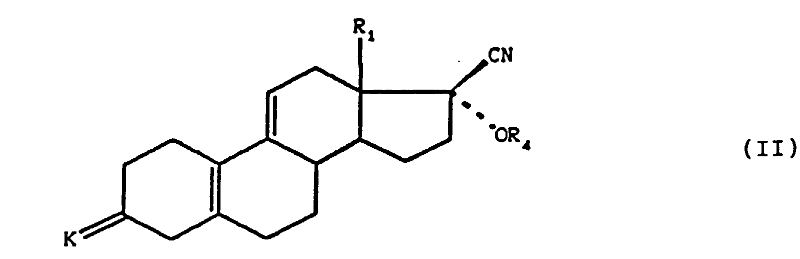 in which R₄ represents a remainder of easily cleavable ether, K represents a protected ketone function in the form of a ketal, thioketal or mixed ketal and R₁ is defined as previously, is treated with an organomagnesium compound reagent of formula (A):
HalMg-CH₂-R₂ (A)
in which Hal represents a halogen atom and R₂ is defined as previously, in order to obtain a compound of formula (III): 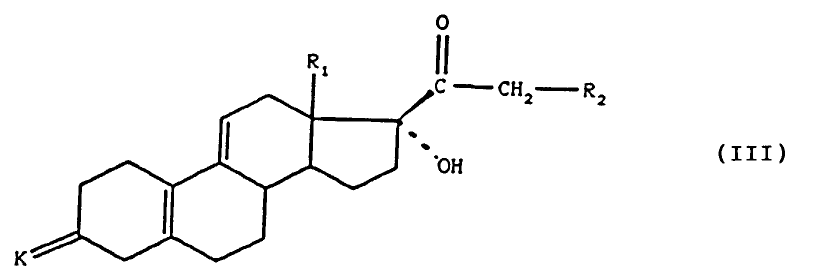 in which R₁, R₂ and K are defined as previously, which is treated with an acylation agent in order to obtain a compound of formula (IV): 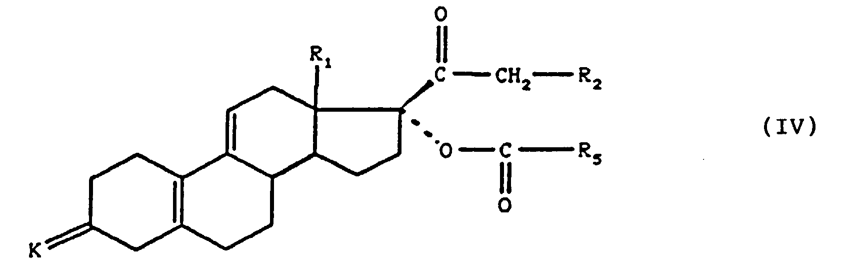 in which R₅ represents an alkyl radical containing 1 to 6 carbon atoms or an aryl radical containing 6 to 10 carbon atoms and R₁, R₂ and K are defined as previously, which is subjected to the action of an appropriate alkylation agent, in order to obtain a compound of formula (V): 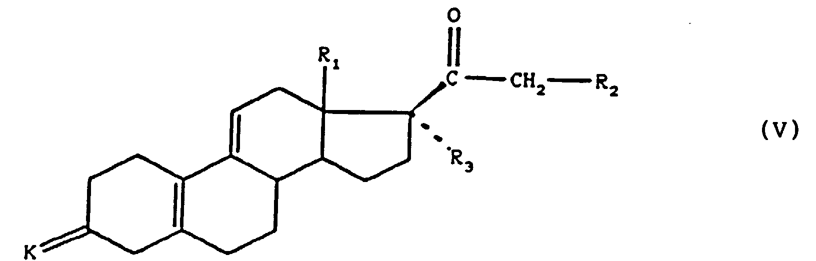 in which R₁, R₂, R₃ and K are defined as previously, which is subjected to an autoxidation reaction, in order to obtain a compound of formula (VI): 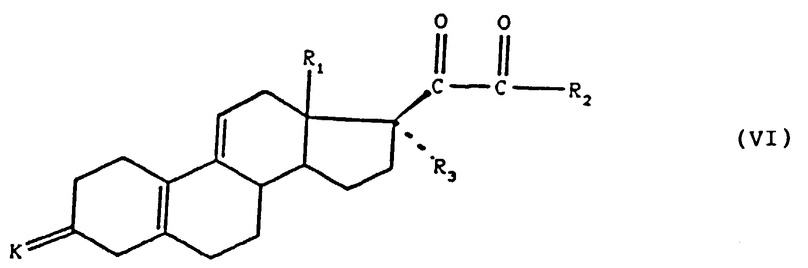 in which R₁, R₂, R₃ and K are defined as previously which is treated with an acid in order to obtain the reconjugated 3-keto compound of formula (VII): 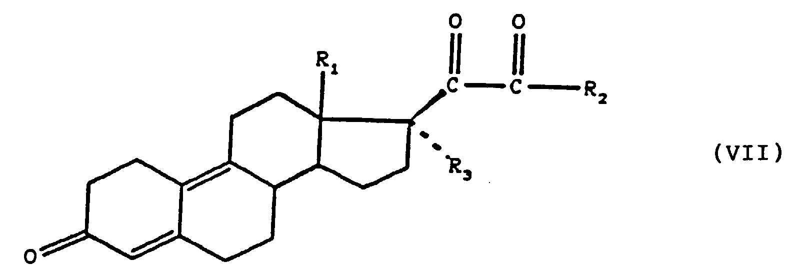 in which R₁, R₂ and R₃ are defined as previously, which is subjected to the action of a regio- and enantioselective reducing agent in order to obtain the expected compound of formula (I).

2. Process according to claim 1, characterized in that a compound of formula (II) is used at the start in which R₄ represents an alkyl radical containing 1 to 6 carbon atoms, a tetrahydropyranyl radical, an aryl radical containing 6 to 12 carbon atoms or a silylated radical.

3. Process according to claim 2, characterized in that a compound of formula (II) is used at the start in which R₄ represents a trialkylsilyl radical and in particular trimethylsilyl and K represents a ketal group and in particular ethylene ketal.

4. Process according to any one of claims 1 to 3, characterized in that the acylation agent is acetic anhydride or acetyl chloride or bromide.

5. Process according to any one of claims 1 to 4, characterized in that the alkylation agent is an alkyl halide or sulphate.

6. Process according to any one of claims 1 to 5, characterized in that the autoxidation reaction is carried out by bubbling through oxygen or air on an enolate in position 20 obtained by action of a strong base on the compound of formula (V).

7. Process according to any one of claims 1 to 6, characterized in that the reduction is carried out by enzymatic route.

8. Process according to claim 7, characterized in that the reduction is carried out using a yeast.

9. Process according to claim 7 or 8, characterized in that the reduction is carried out using a yeast chosen from the class of Hemiascomycetes.

10. Process according to claim 7, 8 or 9, characterized in that the reduction is carried out using Saccharomyces cerevisiae.

11. Process according to any one of claims 1 to 10, characterized in that the following are used at the start: a compound of formula (II) in which R₁ represents a methyl radical, a reagent of formula (A) in which R₂ represents a methyl radical and, as alkylation agent, a methylation agent.

12. As new industrial compounds, the compounds of formula (VIII): 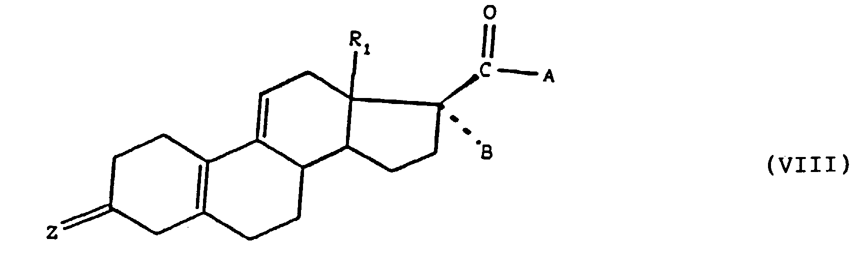 in which R₁ is defined as in claim 1 and either Z represents K, K being defined as in claim 1, A represents -CO-R₂ and B represents R₃, R₂ and R₃ being defined as in claim 1, or Z represents an oxygen atom, A represents -CO-R₂ and B represents R₃, R₂ and R₃ being defined as in claim 1.

13. As new industrial compounds, the compounds of formula (VII) as defined in claim 1.
